(19) **Europäisches Patentamt / European Patent Office / Office européen des brevets**

(11) **EP 4 640 666 A1**

(12) **EUROPEAN PATENT APPLICATION**
published in accordance with Art. 153(4) EPC

(43) Date of publication:
**29.10.2025 Bulletin 2025/44**

(21) Application number: **23906458.7**

(22) Date of filing: **25.10.2023**

(51) International Patent Classification (IPC):
*C07C 53/126* (2006.01)  *C11D 17/06* (2006.01)
*A61Q 19/10* (2006.01)  *C11D 1/04* (2006.01)
*C11D 3/12* (2006.01)  *A61K 8/19* (2006.01)
*A61K 8/26* (2006.01)  *A61K 8/29* (2006.01)
*A61K 8/36* (2006.01)

(52) Cooperative Patent Classification (CPC):
**A61K 8/19; A61K 8/26; A61K 8/29; A61K 8/36;
A61Q 19/10; C07C 53/126; C11D 1/04; C11D 3/12;
C11D 17/06**

(86) International application number:
**PCT/JP2023/038472**

(87) International publication number:
**WO 2024/135084 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.12.2022 JP 2022204425**

(71) Applicant: **Tomoegawa Corporation**
**Tokyo 104-8335 (JP)**

(72) Inventors:
• **IKEYA, Hirotoshi**
**Shizuoka-shi, Shizuoka 421-0192 (JP)**
• **MIYAKE, Rika**
**Shizuoka-shi, Shizuoka 421-0192 (JP)**

(74) Representative: **V.O.**
**P.O. Box 87930**
**2508 DH Den Haag (NL)**

(54) **SOAP COMPOSITE PARTICLES, METHOD FOR PRODUCING SAME, AND COSMETIC**

(57) The problem addressed by the present invention is to provide soap composite particles that can achieve both an improvement in skin feel and durability of function of an inorganic oxide, a method for producing the soap composite particles, and a cosmetic containing the soap composite particles. The soap composite particles of the present invention have a core particle containing a saturated higher fatty acid salt and an inorganic oxide, and an inorganic oxide externally added to the core particle, and are characterized in that the content of the inorganic oxide in the soap composite particles is 5 to 80% by weight, and the shape of the core particle is spherical with a circularity of 0.80 to 1.00.

**Description**

TECHNICAL FIELD

The present invention relates to a soap composite particle, a method for producing same, and a cosmetic.

BACKGROUND ART

Patent Document 1 discloses a metallic soap that can impart high dispersibility or coating property to a powder and improve the anti-caking property, flowability, or feel of the powder, and a method for producing the same. The metallic soap described in Patent Document 1 is characterized in that the internal incorporation rate A, defined as the proportion of the inorganic crystal nucleating agent incorporated inside the metal soap particle, is 30% or more. The internal absorption rate A defined in Patent Document 1 is expressed by the following formula (1).

$$\text{Internal intake rate } A = 100 - (X/X') \times 100 \quad (1)$$

In formula (1), X is the content of inorganic nucleating agent in the metallic soap, X' is the content of inorganic nucleating agent in the ground metallic soap that has passed through a 325 mesh filter, and X and X' are average values obtained by elemental analysis under specified conditions using a scanning electron microscope/energy dispersive X-ray spectroscopy (SEM/EDX). The greater the content of the inorganic crystal nucleating agent that cannot be observed without pulverization, the smaller the value of X relative to X' becomes, and the greater the internal incorporation rate A becomes.

Citation List

Patent Document

Patent Document 1: Patent No. 6729546

SUMMARY OF INVENTION

Technical Problem

Patent Document 1 suggests that when an inorganic crystal nucleating agent is incorporated inside metallic soap particles, the metallic soap particles have a uniform shape and a sharp particle size distribution, and that the solid powder cosmetic preparations in the examples have a pleasant feel. However, Patent Document 1 does not describe or suggest how to achieve both an improvement in skin feel and durability of function of the inorganic oxide.

The present invention has been made in consideration of the above circumstances, and an object of the present invention is to provide soap composite particles that can achieve both an improvement in skin feel and durability of function of an inorganic oxide, a method for producing the same, and a cosmetic containing the soap composite particles.

Solution to Problem

A first aspect of the present invention is soap composite particles comprising: a core particle containing a saturated higher fatty acid salt and an inorganic oxide, and an inorganic oxide externally added to the core particle, characterized in that the content of the inorganic oxide in the soap composite particles is 5 to 80% by weight, and the shape of the core particle is spherical with a circularity of 0.80 to 1.00.

A second aspect is the soap composite particles according to the first aspect, characterized in that the carbon number of the saturated higher fatty acid salt is within the range of 10 to 20.

A third aspect is the soap composite particles according to the first or second aspect, characterized in that the metal forming the saturated higher fatty acid salt is one or more of Li, Na, Al, Mg, Ca, Zn, and Ba.

A fourth aspect is the soap composite particles according to any one of the first to third aspects, characterized in that the content of the inorganic oxide in the core particle is 5 to 75% by weight, and the amount of the inorganic oxide added externally per 100 parts by weight of the core particle is 0.01 to 5.0 parts by weight.

A fifth aspect is the soap composite particles according to any one of the first to fourth aspects, characterized in that the inorganic oxide is one or more of zinc oxide, magnesium oxide, titanium dioxide, mica, talc, kaolin, sericite, silica, iron oxide, alumina, zirconia, chromium oxide, and zeolite.

A sixth aspect is the soap composite particles according to any one of the first to fifth aspects, characterized in that the volume-based median diameter of the core particle is greater than 3 $\mu$m and less than 20 $\mu$m.

A seventh aspect is the soap composite particles according to any one of the first to sixth aspects, characterized in that the inorganic oxide is subjected to a hydrophobic treatment.

An eighth aspect is the soap composite particles according to the seventh aspect, characterized in that the hydrophobic treatment is any one of a silane coupling treatment, a silicone oil treatment, and a fatty acid salt treatment.

A ninth aspect is the soap composite particles according to any one of the first to eighth aspects, characterized in that the inorganic oxide has an average particle size of 0.01 to 0.8 $\mu$m.

The tenth aspect is a method for producing soap composite particles according to any one of the first to ninth aspects, comprising: a kneading step of melting and kneading a mixed powder of a saturated higher fatty acid salt and an inorganic oxide; a pulverizing step of pulverizing the solid kneaded product obtained in the kneading step; a spheroidization step of heating the particles obtained in the pulverizing step to form them into spheroids; and an external addition step of externally adding an inorganic oxide to core particles obtained in the spheroidization step.

An eleventh aspect is a cosmetic comprising the soap composite particles according to any one of the first to ninth aspects.

Advantageous Effects of Invention

According to the present invention, it is possible to provide soap composite particles that can both achieve an improvement in the skin feel and durability of function of an inorganic oxide, and a method for producing the same. Furthermore, according to the present invention, it is possible to provide a cosmetic containing the soap composite particles, and it is expected that such a cosmetic will achieve a favorable skin feel while maintaining durability of function as soap.

DESCRIPTION OF EMBODIMENTS

The present invention will be described below using an embodiment. However, the present embodiment is specifically described to provide a better understanding of the gist of the invention, and does not limit the present invention unless otherwise specified. Other embodiments in which those skilled in the art appropriately have replaced the configurations of the following embodiments are also included in the scope of the present invention.

The present invention relates to soap composite particles, which have a core particle containing a saturated higher fatty acid salt and an inorganic oxide, and an inorganic oxide externally added to the core particle.

<Saturated higher fatty acid salt>

The saturated higher fatty acid salt may be a salt of an alkali metal such as sodium or potassium, or a salt of a metal other than the alkali metal. Saturated higher fatty acid salts may be produced by a conventional method using saturated higher fatty acids or their alkali metal salts and inorganic metal salts as raw materials, or commercially available products may be used. The saturated higher fatty acid salt may be one type or a mixture of two or more types.

Suitable saturated higher fatty acids for use in the saturated higher fatty acid salts include hexanoic acid, caproic acid (n-hexanoic acid), heptanoic acid, octanoic acid, caprylic acid (n-octanoic acid), nonanoic acid, decanoic acid, capric acid (n-decanoic acid), lauric acid, myristic acid, palmitic acid, stearic acid, arachic acid, behenic acid, and montanic acid.

Because saturated higher fatty acids do not have double bonds (unsaturated bonds) in the hydrocarbon chain, they are highly chemically stable, less susceptible to deterioration due to oxygen or ultraviolet light, and tend to maintain excellent quality. The saturated higher fatty acid salt may be a branched chain, but the branched side chain preferably contains 2 or less carbon atoms, more preferably 1 or less carbon atoms. Straight-chain saturated higher fatty acid salts are particularly preferred.

By using straight-chain saturated higher fatty acid salts, the hydrocarbon chain does not have a double bond (unsaturated bond) or a side chain, so the structure is stable and the methyl groups at the molecular ends are neatly arranged on the surface of the soap composite particle. Furthermore, since the melting point is high, it is unlikely to become liquid at room temperature (5 to 35°C), and excellent quality is likely to be maintained.

The number of carbon atoms of the saturated higher fatty acid salt is not particularly limited, but may be, for example, within the range of 8 to 30, preferably within the range of 10 to 20, more preferably within the range of 10 to 18, and particularly preferably within the range of 12 to 18. When the number of carbon atoms is equal to or greater than the lower limit, the saturated higher fatty acid salt exhibits hydrophobicity and is less susceptible to the effects of moisture. When the number of carbon atoms is equal to or less than the upper limit, the molecules are easily aligned, the structure is stable, the melting point is high, and the durability is improved.

The metal salt used in the saturated higher fatty acid salt is not particularly limited, but examples thereof include alkali metal salts, alkaline earth metal salts, and transition metal salts. Examples of metals that form the saturated higher fatty acid salt include one or more of Li, Na, Al, Mg, Ca, Zn, and Ba, and preferably one or more of Zn, Mg, Ca, and Al, and more preferably one or more of Zn, Mg, and Ca. These compounds form stable structures in the soap itself. Among these, it is preferable to select a compound that has little effect on the human body and is insoluble in water.

In particular, in applications in which a composition containing soap composite particles is applied to the skin, when a saturated higher fatty acid salt is used, the molecular ends form a uniform hydrophobic surface, which makes the composition less susceptible to the effects of moisture in the air and moisture in body fluids such as sweat, improving the skin feel. Compositions that are applied to the skin can be used as cosmetics, medicines, other pharmaceuticals, and the like.

The saturated higher fatty acid salt preferably contains no polar functional groups such as hydroxyl groups or epoxy groups, except for a carboxylate ion group ($-COO^-$) at one end. This improves the hydrophobicity. The saturated higher fatty acid salt is preferably a non-cyclic compound represented by the general formula $(C_{n-1}H_{2n-1}COO)_mM$, and more preferably a straight-chain compound represented by the general formula $[CH_3(CH_2)_{n-2}COO]_mM$. In these general formulas, n represents the number of carbon atoms, and M represents a metal.

When the metal M is polyvalent, that is, when m is 2 or more, the carbon number n of the m saturated higher fatty acids may be the same or different. When the carbon numbers n of the m saturated higher fatty acids are different from one another, the difference between the maximum carbon number and the minimum carbon number is preferably 4 or less, and more preferably 2 or less. In order for the molecular ends of the saturated higher fatty acid salt to form a more uniform hydrophobic surface, it is preferable that the number of carbon atoms of each saturated higher fatty acid constituting the saturated higher fatty acid salt is the same.

<Inorganic oxide>

The soap composite particles of the embodiment have an inorganic oxide added internally to the core particles and an inorganic oxide added externally to the core particles. The core particle is formed by adding an inorganic oxide to a saturated higher fatty acid salt. The inorganic oxide added to the core particle may be embedded within the core particle. The inorganic oxide added to the outside of the core particle preferably covers at least a part of the outer surface of the core particle.

Examples of inorganic oxides include inorganic particles of metal oxides, metal nitrides, silicates, sulfates, carbonates, phosphates, and the like. Specific examples of inorganic oxides include one or more of zinc oxide, magnesium oxide, titanium dioxide, mica, talc, kaolin, sericite, silica, iron oxide, alumina, zirconia, chromium oxide, and zeolite.

The inorganic oxide particles are preferably functional particles. The functionality is preferably such that when the soap composite particles are used, the inorganic oxide particles act on an object together with the saturated higher fatty acid salt. Specific examples of functionality include, but are not limited to, ultraviolet reflectivity, sunscreen, hiding properties as a pigment, anti-inflammatory properties against inflammation, X-ray absorption, solid lubricity, release properties, antibacterial properties, electrical insulation properties, and the like.

The content of the inorganic oxide in the soap composite particles is preferably from 5 to 80% by weight, more preferably from 20 to 55% by weight. The content of the inorganic oxide in the core particle is preferably from 5 to 75% by weight, and more preferably from 20 to 50% by weight. When the content of the inorganic oxide is equal to or higher than the lower limit, the exposed area of the inorganic oxide added to the core particle is increased, improving the durability of function of the inorganic oxide. When the content of the inorganic oxide is equal to or less than the upper limit, the salt of the saturated higher fatty acid and the inorganic oxide are easily kneaded, and the uniformity of the core particles is improved.

The amount of the inorganic oxide added externally relative to 100 parts by weight of the core particles is preferably 0.01 to 5.0 parts by weight, and more preferably 0.05 to 2.0 parts by weight. When the amount of the inorganic oxide added externally is equal to or greater than the lower limit, the amount of the inorganic oxide present on the surface of the core particles increases, improving the durability of function of the inorganic oxide. When the amount of the inorganic oxide added externally is equal to or less than the upper limit, the lubricity and tactile feel characteristic of the saturated higher fatty acid salt become more prevalent.

The inorganic oxide externally added to the core particle may be the same type of inorganic oxide as the inorganic oxide internally added to the core particle, or may be a different inorganic oxide. The inorganic oxide internally added to the core particle may be one type or two or more types. When two or more kinds of inorganic oxides are internally added to the core particle, the content may be the total amount of the two or more kinds. The inorganic oxide externally added to the core particle may be one type or two or more types. When two or more kinds of inorganic oxides are externally added to the core particle, the amount of external addition may be the total amount of the two or more kinds.

The inorganic oxide externally added to the core particle may function as a fluidity imparting agent. In this case, the fluidity imparting agent exerts a bearing effect, increasing the fluidity of the soap composite particles and improving the skin feel. As the inorganic oxide to be externally added to the core particle, particles that function as a fluidity imparting agent and particles having other functions may be used in combination.

The inorganic oxide may be coated, for example, it may be subjected to a hydrophobization treatment. Examples of the hydrophobization treatment include a silane coupling treatment using a silane coupling agent, a silicone oil treatment using a silicone oil such as a long-chain alkylsilane, and a fatty acid salt treatment using a fatty acid salt. In the silicone oil treatment, modified silicone oil may be used.

In the case of the silane coupling treatment or silicone oil treatment, siloxane bonds are formed and fixed on the inorganic oxide surface by hydrolysis of the silane coupling agent or silicone oil and a dehydration condensation reaction, and the inorganic oxide is made hydrophobic. Therefore, inorganic oxides have high dispersibility even when kneaded into soap made of a hydrophobic salt of a higher saturated fatty acid at high temperatures.

In the case of fatty acid salt treatment, the composition of the fatty acid salt is similar to that of higher saturated fatty acid salts used in soap. Therefore, the inorganic oxide surface-treated with the fatty acid salt has high dispersibility in the soap into which it is kneaded, and has high adhesion to the soap. When the fatty acid salt used in the fatty acid salt treatment corresponds to the above-mentioned higher saturated fatty acid salt, its mass may be excluded from the mass of the inorganic oxide and added to the mass of the higher saturated fatty acid salt.

The average particle size of the inorganic oxide is, for example, within the range of 0.01 to 0.8 μm, and preferably within the range of 0.01 to 0.5 μm. When the average particle size of the inorganic oxide is equal to or greater than the lower limit, the inorganic oxide particles are less likely to aggregate and are more likely to disperse. When the average particle size of the inorganic oxide is equal to or less than the upper limit, the inorganic oxide is easily added to the core particles, and even when externally added, it is difficult for the inorganic oxide to fall off from the soap composite particles.

<Core particle>

The core particle preferably has a spherical shape with a circularity of 0.80 to 1.00, and more preferably have a circularity of 0.90 to 1.00. The saturated higher fatty acid salt in the core particle acts as a lubricant, making them easier to roll without resistance. When the circularity of the core particle is equal to or higher than the lower limit, the particle tends to roll and slide without resistance. For example, when a composition containing soap complex particles is applied onto the skin using the finger pads of the hand, the particles can roll easily and provide a good effect on the skin. The theoretical maximum circularity is 1, which corresponds to the case where the particle image is a perfect circle.

The circularity can be calculated by analyzing a particle image, measuring the area A and circumference C of the particle image, and using the following formula where D is the diameter of a circle (equivalent circle diameter) equal to the area A of the particle image and $\pi$ is the circularity constant.

$$(\text{Circularity}) = \pi D/C$$

The circularity of the particles may be measured, for example, using a flow-type particle image analyzer that measures particle shape, particle size distribution, particle number, etc. by two-dimensional image analysis of a particle group. Alternatively, the circularity may be calculated by observing the surface of a particle from multiple fields of view using an electron microscope and performing stereoscopic image analysis.

The volume-based median diameter of the core particle is preferably more than 3 μm and less than 20 μm, more preferably more than 5 μm and less than 15 μm, and even more preferably more than 7 μm and less than 10 μm. If the volume-based median diameter of the core particle is above the lower limit, the particles will have a small cohesive force and will be less likely to form agglomerates. When the volume-based median diameter of the core particle is less than the upper limit, the surface area per unit weight becomes large, the exposed area of the inorganic oxide added to the core particles becomes large, and the durability of function of the inorganic oxide is improved.

In applications in which a composition containing soap composite particles is applied to the skin, if the volume-based median diameter of the core particle is above the lower limit, the particles spread easily on the skin, providing a uniform feel when spread. When the volume-based median diameter of the core particle is less than the upper limit, the roughness caused by the particle size is hardly felt on the fingertips, and the feel on the skin is improved.

<Method for producing soap composite particles>

The present invention relates to a method for producing soap composite particles, which includes, for example, a kneading step of melting and kneading a mixed powder of a saturated higher fatty acid salt and an inorganic oxide, a pulverizing step of pulverizing the solid kneaded product, a spheroidization step of heating the particles to make them spherical, and an external addition step of externally adding the inorganic oxide to core particles.

Prior to the kneading step, the method may include a measuring step of measuring the saturated higher fatty acid salt and the inorganic oxide to be used in the preparation of the core particles, and a mixing step of stirring and mixing the measured saturated higher fatty acid salt and the inorganic oxide. The particles can be mixed using any mixing device, including, but not limited to, a double cone mixer, a V-type mixer, a drum mixer, a Super Mixer, a Henschel mixer, a Nauta mixer, or the like.

Although it is possible to mix a portion of the raw materials during the kneading step, it is preferred to premix at least a portion of the raw materials prior to the kneading step. By carrying out the premixing, it is advantageous to control the composition of the saturated higher fatty acid salt and inorganic oxide, the size and shape of the particles, and the like.

The kneading step is preferably carried out under conditions where the salt of the saturated higher fatty acid is melted or softened by heating. As a result, the inorganic oxide mixed with the saturated higher fatty acid salt is added internally to the saturated higher fatty acid salt. The kneader may be a batch type kneader or a continuous type kneader. The kneading machine is not particularly limited, but examples thereof include an open roll, a kneader, a pressure kneader, a Banbury mixer, a single screw extruder, and a twin screw extruder. The heating temperature in the kneading step can be appropriately set depending on the melting point of the saturated higher fatty acid salt, and may be, for example, about 80 to 250°C, or about 100 to 200°C.

The pulverizing step is a step of pulverizing the solid kneaded product obtained by cooling the kneaded product obtained in the kneading step. For this reason, it is preferable to carry out a cooling step between the kneading step and the pulverizing step. The residual heat may be absorbed by a cooling member in contact with the kneaded material, for example, by passing the molten kneaded material through a cooling roll. A cooling member such as a cooling roll may have a cooling medium such as cooling water therein. In the cooling step, heat can be dissipated from the kneaded material by air cooling, blowing air, or the like.

The pulverizing step may be carried out in multiple stages, such as a coarse pulverizing step and a fine pulverizing step. The device used for coarsely pulverizing the particles is not particularly limited, but a crusher, a hammer mill, a feather mill, a cutter mill, or the like can be used. The device used for finely pulverizing the particles is not particularly limited, but a jet mill, a counter jet mill, a high-speed rotor rotary mill, etc. can be used.

When the solid kneaded product is pulverized to a desired particle size, the particle size may be adjusted by a classification step, if necessary. In the classification step, for example, an elbow jet classifier of an inertial classification type, a Microplex of a centrifugal classification type, an airflow type classifier, or the like can be used. Particles that are coarser than the desired particle size may be returned to the pulverizing process for further pulverizing. Particles finer than the desired particle size may be recycled by returning them to the kneading step, depending on the composition.

The spheroidization step is a step in which the particles obtained in the pulverizing step are spheroidized by heating. The spheroidization device is not particularly limited, but examples thereof include a device for mechanically adjusting the particle shape, such as an impact spheroidization device, a device for adjusting the particle shape by dissolving the binder and removing the solvent, such as a spray dryer, and a device for adjusting the particle shape by heating in a medium or using hot air. The spheroidization step produces spherical core particles in which the inorganic oxide is internally added to the saturated higher fatty acid salt. If necessary, a classification step can be carried out after the core particle spheroidization step.

The external addition step involves mixing the inorganic oxide with core particles to externally add the inorganic oxide to the core particles. In the step of externally adding the inorganic oxide, a mixing device such as a Super Mixer or a Henschel mixer may be used, or a powder processing device such as a Mechano Mill may be used.

<Soap composite particles>

The soap composite particles of the present invention have functionality due to the internally or externally added inorganic oxide in addition to the functionality of the saturated higher fatty acid salt, and therefore can be used as a variety of functional materials. The uses of the soap composite particles are not particularly limited, but examples thereof include use as a feel improver, lubricant, release agent, anti-caking agent, cosmetic raw material, colorant, processing aid, dispersant, additive, etc.

<Method of analyzing soap composite particles>

As described above, the soap composite particle of the present invention has an inorganic oxide added internally to the core particle and an inorganic oxide added externally to the core particle. Since the core particle is mainly composed of a saturated higher fatty acid salt, it is possible to separate the saturated higher fatty acid salt from the inorganic oxide. This makes it possible to evaluate the amount of internal addition and the amount of external addition even if the inorganic oxide internally added to the core particle and the inorganic oxide externally added to the core particle are the same substance.

A specific example of a method for separating the core particle from the external additive (externally added inorganic oxide) is a method in which a pre-weighed amount of soap composite particle powder is added to a dispersion medium, the resulting dispersion is then inserted into the probe of an ultrasonic homogenizer, and ultrasonic waves are applied. This allows the external additives that have fallen off the core particles to be suspended in the dispersion medium. The core particles are allowed to settle by standing or centrifuging, and then the supernatant liquid in which the external additives are suspended can be extracted and separated by a liquid separation operation or the like.

The dispersion medium is preferably an aqueous solution prepared by dissolving a surfactant in water in order to suppress aggregation of the powder. Since the saturated higher fatty acid salt is insoluble in water, the added inorganic oxide can maintain the state of being enclosed in the core particle.

The amount of core particles from which the external additives have been separated can be determined by repeatedly washing the sediment with a solvent and filtering it using filter paper capable of collecting the core particles, and then drying the sediment and weighing it. The collection performance of the filter paper is preferably such that it can retain particles of, for example, 1 μm or larger. The amount of the external additive may be calculated by subtracting the mass of the core particles obtained by weighing from the mass of the soap composite particles.

The components and composition of the external additives contained in the supernatant can be analyzed by, for example, X-ray fluorescence analysis (XRF) of the supernatant.

The components and composition of the inorganic oxide added to the core particles can be analyzed, for example, by X-ray fluorescence analysis (XRF) of the core particles obtained as a precipitate.

<Cosmetic containing soap composite particles>

The cosmetic of the present invention contains the above-mentioned soap composite particles. Examples of cosmetics containing soap composite particles include makeup cosmetics such as foundation, liquid foundation, eye shadow, blush, face powder, concealer, blush, eyebrow powder, and highlighter; cleansing products such as cleansing agents and facial washes; skin care cosmetics such as massage cream, moisture cream, and milk lotion; body care cosmetics such as bath additives, sunscreen, sunscreen cream, and deodorant spray; basic cosmetics such as makeup base; and hair care cosmetics such as shampoo, rinse, hair liquid, and hair color. These cosmetics can be prepared by mixing and processing various raw materials in proportions suitable for the intended use.

In the cosmetic of the present invention, the above-mentioned soap composite particles can be contained in an amount of 0.5% by mass to 60% by mass based on the total amount of the cosmetic. The soap composite particles can be preferably contained in the range of 1% by mass to 40% by mass, and more preferably 2% by mass to 30% by mass, based on the total amount of the cosmetic. If the content of the soap composite particles is less than 0.5% by mass, it is difficult to feel the improving effect on skin feel when using the cosmetic, and if it exceeds 60% by mass, it is not possible to expect an effect commensurate with the amount blended, and problems are likely to arise in maintaining the quality and stability of the cosmetic.

In the cosmetic of the present invention, in addition to the above-mentioned soap composite particles, various ingredients that are conventionally used in cosmetics can be blended. Examples of the components include pigments, surface-treated pigments, ultraviolet absorbers, physiologically active ingredients, oils, surfactants, fluorine compounds, resins, thickeners, preservatives, fragrances, moisturizers, salts, solvents, antioxidants, chelating agents, neutralizing agents, pH adjusters, and insect repellents. These ingredients can be blended in amounts that do not impair the effects of the cosmetic composition of the present invention.

The present invention has been described above based on a preferred embodiment, but the present invention is not limited to the above-mentioned embodiment, and various modifications are possible without departing from the gist of the present invention.

EXAMPLES

Examples of the present invention will be described below, but the present invention is not limited to these examples.

<Production of soap composite particles>

By carrying out each of the following steps, soap composite particles in which an inorganic oxide is externally added to the core particle containing a saturated higher fatty acid salt and an inorganic oxide were produced.

(Mixing step)

As shown in the "Saturated higher fatty acid salt" and "Inorganic oxide internal addition amount" in Tables 1 to 4, the saturated higher fatty acid salt and the inorganic oxide were each weighed, placed in a 20 L Henschel mixer (FM20 manufactured by Nippon Coke and Engineering Co., Ltd.), and mixed by stirring to obtain a mixed powder.

(Kneading step)

The mixed powder obtained in the mixing step was heated to 120 to 140° C and melt-kneaded in a twin-screw kneader (PCM-30 manufactured by Ikegai Corporation). The molten mixture discharged from the kneader was passed through a cooled rolling mill roll to obtain a plate-like solid mixture.

(Pulverizing step)

The plate-like solid kneaded product obtained in the mixing step was coarsely pulverized in a hammer mill to obtain coarsely pulverized products of 0.5 to 3.0 mm. The coarsely pulverized material was finely pulverized in a jet mill (ULTRA SONIC JET MILL I-2, manufactured by Nippon Pneumatic Mfg. Co., Ltd.) to obtain a finely pulverized material having an average particle size by volume of 3 to 20 $\mu$m.

(Classification step)

The finely pulverized material obtained in the pulverizing step was classified using an airflow classifier (DS2UR, manufactured by Nippon Pneumatic Mfg. Co., Ltd.) to remove particles of 3 $\mu$m or less.

(Spheroidization step)

The classified powder obtained in the classification step was treated in a hot air spheroidization device (Meteor Rainbow MR, manufactured by Nippon Pneumatic Mfg. Co., Ltd.) heated to 360° C to be spheroidized.

(External addition step)

To the spherical core particles obtained in the spheroidization step, inorganic oxide was externally added as shown in the "Inorganic Oxide External Addition Amount" in Tables 1 to 4, to obtain soap composite particles.

<Structure of core particle>

The spherical core particles obtained in the spheroidization step were used as samples, and the structure of the core particles was measured by the following method. In addition, the core particles separated from the external additives using the above-mentioned method for analyzing soap composite particles can also be used as a sample.

(Circularity)

The circularity of the core particles was measured using a flow type particle image analyzer.

(Volume-based median diameter)

The median diameter (D50) was determined by measuring the volume distribution using a Coulter counter utilizing the electrical change occurring when the core particles pass through an aperture tube.

<Skin feel evaluation method>

An earpick-sized amount of soap composite particle powder was placed on the wrist of one arm, and the powder was gently rolled over the skin with the middle finger of the other hand in a circular motion of 4 cm in diameter for 15 seconds, and the feel was evaluated using the middle finger and wrist. A panel of four people (two adult males and two adult females) evaluated the usability by categorizing it into specific items, assigning scores to each item, and conducting a sensory assessment. The evaluation items and evaluation criteria are as follows:

(Evaluation items)

Spreadability: Whether the powder spreads well on the skin when applied.
Adhesive: Whether the powder fills into pores well when applied.
Smoothness: When applying powder, does it feel smooth on the skin after application?

Lightness: When applying the powder, does it feel light in weight after application?
Lasting power: Whether the makeup lasts well when you apply the powder and rub it with your fingers.
Softness: When applying powder, whether the powder feels soft after application.

(Score for each item)

5: Very good.
4: Excellent.
3: Normal.
2: Inferior.
1: Very poor. (Evaluation criteria)
A: The total score is between 24 and 30.
B: The total score is 18 or more but less than 24.
C: The total score is 12 or more but less than 18.
D: The total score is between 6 and 12.

<Method for evaluating the durability of function of titanium dioxide>

As a function of titanium dioxide, the SPF (Sun Protection Factor) value, which is an index of the ultraviolet ray protection function, was measured, indicating how much protection it provides against UVB among ultraviolet rays. Specifically, a powder of the soap composite particles was applied to a polymethyl methacrylate (PMMA) plate and dried, and the SPF value of the prepared sample was measured using an SPF analyzer. The evaluation value was the average of the measurements at five points. A practically acceptable level of UV protection is SPF 20 or higher.

(Evaluation criteria)

A: The average SPF is 40 or higher.
B: The average SPF is 30 or more and less than 40.
C: The average SPF is 20 or more and less than 30.
D: The average SPF is less than 20.

<Method for evaluating the durability of function of zinc oxide>

As a function of zinc oxide, the UVAPF (Ultraviolet A Protection Factor) value, which is an index of the ultraviolet protection function of the zinc oxide, was measured, indicating how much protection it provides against UVA among ultraviolet rays. Specifically, a powder of the soap composite particles was applied to a polymethyl methacrylate (PMMA) plate and dried, and the UVAPF value of the prepared sample was measured using an SPF analyzer. The evaluation value was the average of the measurements at five points.

(Evaluation criteria)

A: The average UVAPF value is 16 or higher.
B: The average UVAPF value is 8 or greater but less than 16.
C: The average UVAPF value is 4 or more and less than 8.
D: The average UVAPF value is 2 or more and less than 4.

<Method for evaluating the durability of function of zeolite>

The antibacterial effect of zeolite was evaluated. The test species used were Escherichia coli, Staphylococcus aureus, Pseudomonas aeruginosa, Aspergillus niger, and Candida albicans, which are standard strains used in testing the preservative effectiveness of cosmetics, as well as Propionibacterium acnes and Malassezia fungi, which are normal flora found on human skin.

The evaluation sample was prepared by adding 0.1 g of soap composite particles to 1 mL of a test bacterial solution containing an inoculated bacterial count of $1.0 \times 10^5$ CFU (Colony Forming Unit)/mL. The blank sample consisted of 1 mL of the test bacteria solution alone. After 24 hours, the viable cell count was measured by a standard method, and the antibacterial activity was evaluated based on the survival rate of the bacteria according to the following evaluation criteria.

(Evaluation criteria)

A: The survival rate of bacteria is between 0% and 20%.
B: The survival rate of the bacteria is more than 20% and less than 70%.
C: The survival rate of the bacteria is more than 71% and less than 95%.
D: The survival rate of the bacteria is more than 95% and less than 100%.

<Overall evaluation>

The overall evaluation was based on the evaluation of the skin feel and the evaluation of the durability of function, and was made according to the following evaluation criteria.

(Evaluation criteria)

A: The evaluation of the skin feel and the evaluation of the durability of function were both A.
B: At least one of the evaluations is B, and the evaluations of C and D are not included.
C: At least one of the evaluations is C, and no evaluation is D.
D: At least one of the evaluations is D.

<Evaluation results>

The evaluation results are shown in Tables 1 to 4. In the "Saturated higher fatty acid salts" column, fatty acid salts are displayed by combining the name of the fatty acid with the element symbol of the metal, for example, "Ca stearate" represents "calcium stearate." In some comparative examples, an unsaturated fatty acid salt was used.

Stearic acid (18 carbon atoms), lauric acid (12 carbon atoms), palmitic acid (16 carbon atoms), caprylic acid (8 carbon atoms), and behenic acid (22 carbon atoms) are straight-chain saturated fatty acids. In addition, oleic acid (18 carbon atoms, 1 double bond) and linoleic acid (18 carbon atoms, 2 double bonds) are straight-chain unsaturated fatty acids.

The weight parts in Tables 1 to 4 were set so that the total of the saturated higher fatty acid salt and the internally added amount of the inorganic oxide was 100 weight parts. Therefore, the amount (parts by weight) of the inorganic oxide added internally is equal to the content (% by weight) of the inorganic oxide in the core particles.

In the "Total (wt. %)" column of "Inorganic oxide" in Tables 1 to 4, the ratio of the total amount of inorganic oxide (the sum of the amount added internally and the amount added externally) to the total amount of soap composite particles (the sum of the saturated higher fatty acid salt and inorganic oxide) is calculated and shown to one decimal place.

In the "Hydrophobization Treatment" column of Tables 1 to 4, the inorganic oxide that was not hydrophobized is indicated as "None," the silane coupling treatment is indicated as "SiCp," the silicone oil treatment is indicated as "SiOi," and the fatty acid salt treatment is indicated as "FAS."

[Table 1]

| | | Example 1 | Example 2 | Example 3 | Example 4 | Example 5 | Example 6 | Example 7 | Example 8 |
|---|---|---|---|---|---|---|---|---|---|
| Saturated higher fatty acid salt (parts by weight) | Calcium stearate | 70 | 70 | 70 | 70 | 70 | 70 | 70 | 70 |
| | Zinc laurate | | | | | | | | |
| | Sodium palmitate | | | | | | | | |
| | Calcium caprylate | | | | | | | | |
| | Sodium behenate | | | | | | | | |
| | Sodium oleate | | | | | | | | |
| | Calcium linoleate | | | | | | | | |
| Inorganic oxide internal addition amount (parts by weight) | Titanium dioxide | 30 | 30 | 30 | 30 | 30 | 30 | 30 | 30 |
| | Zinc oxide | | | | | | | | |
| | Zeolite | | | | | | | | |
| Inorganic oxide external addition amount (parts by weight) | Titanium dioxide | 1.5 | 1.5 | 1.5 | 1.5 | 1.5 | 1.5 | 1.5 | 1.5 |
| | Zinc oxide | | | | | | | | |
| | Zeolite | | | | | | | | |
| Inorganic oxide Total (weight%) | | 31.0 | 31.0 | 31.0 | 31.0 | 31.0 | 31.0 | 31.0 | 31.0 |
| Structure of core particle | Circularity | 0.95 | 0.95 | 0.95 | 0.95 | 0.95 | 0.95 | 0.95 | 0.95 |
| | D50 ($\mu$m) | 9 | 9 | 9 | 9 | 9 | 2 | 4 | 6 |
| Structure of inorganic oxide | Average particle size (µm) | 0.3 | 0.3 | 0.3 | 0.005 | 0.1 | 0.3 | 0.3 | 0.3 |
| | Hydrophobic treatment | None | FAS | SiOi | SiCp | SiCp | SiCp | SiCp | SiCp |
| Evaluation | Skin feel | △ | ◎ | ○ | ○ | ◎ | △ | ○ | ◎ |
| | Durability of function | △ | ○ | ○ | △ | ◎ | ◎ | ◎ | ◎ |
| | Overall | △ | ○ | ○ | △ | ◎ | △ | ○ | ◎ |

[Table 2]

| | | Comparative Example 1 | Example 9 | Comparative Example 2 | Example 10 | Example 11 | Example 12 | Example 13 | Comparative Example 3 |
|---|---|---|---|---|---|---|---|---|---|
| Saturated higher fatty acid salt (parts by weight) | Calcium stearate | 70 | 70 | 97 | 93 | 93 | 70 | 70 | |
| | Zinc laurate | | | | | | | | |
| | Sodium palmitate | | | | | | | | |
| | Calcium caprylate | | | | | | | | |
| | Sodium behenate | | | | | | | | |
| | Sodium oleate | | | | | | | | |
| | Calcium linoleate | | | | | | | | 70 |
| Inorganic oxide internal addition amount | Titanium dioxide | 30 | 30 | 3 | 7 | 7 | | | 30 |
| | Zinc oxide | | | | | | | 30 | |
| | Zeolite | | | | | | 30 | | |
| Inorganic oxide external addition amount | Titanium dioxide | 1.5 | 1.5 | 0 | 0.005 | 0.05 | | | 1.5 |
| | Zinc oxide | | | | | | | 1.5 | |
| | Zeolite | | | | | | 1.5 | | |
| Inorganic oxide Total (weight%) | | 31.0 | 31.0 | 3.0 | 7.0 | 7.0 | 31.0 | 31.0 | 31.0 |
| Structure of core particle | Circularity | 0.7 | 0.85 | 0.95 | 0.95 | 0.95 | 0.95 | 0.95 | 0.95 |
| | D50 ($\mu$m) | 9 | 9 | 9 | 9 | 9 | 9 | 9 | 9 |
| Structure of inorganic oxide | Average particle size (µm) | 0.3 | 0.3 | 0.3 | 0.3 | 0.3 | 0.3 | 0.3 | 0.3 |
| | Hydrophobic treatment | SiCp | SiCp | SiCp | SiCp | SiCp | SiCp | SiCp | SiCp |
| Evaluation | Skin feel | × | ○ | △ | △ | ○ | ◎ | ◎ | × |
| | Durability of function | ◎ | ◎ | × | △ | ○ | ◎ | ◎ | △ |
| | Overall | × | ○ | × | △ | ○ | ◎ | ◎ | × |

[Table 3]

| | | Comparative Example 4 | Example 14 | Example 15 | Example 16 | Example 17 | Example 18 | Example 19 | Example 20 |
|---|---|---|---|---|---|---|---|---|---|
| Saturated higher fatty acid salt (parts by weight) | Calcium stearate | | | | | | 70 | 28 | 22 |
| | Zinc laurate | | | | | 70 | | | |
| | Sodium palmitate | | | | 70 | | | | |
| | Calcium caprylate | | | 70 | | | | | |
| | Sodium behenate | | 70 | | | | | | |
| | Sodium oleate | 70 | | | | | | | |
| | Calcium linoleate | | | | | | | | |
| Inorganic oxide internal addition amount | Titanium dioxide | 30 | 30 | 30 | 30 | 30 | 30 | 72 | 78 |
| | Zinc oxide | | | | | | | | |
| | Zeolite | | | | | | | | |
| Inorganic oxide external addition amount | Titanium dioxide | 1.5 | 1.5 | 1.5 | 1.5 | 1.5 | 1.5 | 1.5 | 1.5 |
| | Zinc oxide | | | | | | | | |
| | Zeolite | | | | | | | | |
| Inorganic oxide Total (weight%) | | 31.0 | 31.0 | 31.0 | 31.0 | 31.0 | 31.0 | 72.4 | 78.3 |
| Structure of core particle | Circularity | 0.95 | 0.95 | 0.95 | 0.95 | 0.95 | 0.95 | 0.95 | 0.95 |
| | D50 (μm) | 9 | 9 | 9 | 9 | 9 | 9 | 9 | 9 |
| Structure of inorganic oxide | Average particle size (μm) | 0.3 | 0.3 | 0.3 | 0.3 | 0.3 | 0.3 | 0.3 | 0.3 |
| | Hydrophobic treatment | SiCp | SiCp | SiCp | SiCp | SiCp | SiCp | SiCp | SiCp |
| Evaluation | Skin feel | × | ○ | ○ | ◎ | ◎ | ◎ | ◎ | ○ |
| | Durability of function | △ | ○ | ○ | ◎ | ◎ | ◎ | ◎ | ◎ |
| | Overall | × | ○ | ○ | ◎ | ◎ | ◎ | ◎ | ○ |

[Table 4]

| | | Example 21 | Example 22 | Comparative Example 5 | Example 23 | Example 24 | Example 25 | Example 26 | Example 27 |
|---|---|---|---|---|---|---|---|---|---|
| Saturated higher fatty acid salt (parts by weight) | Calcium stearate | 70 | 30 | 18 | 70 | 70 | 70 | 70 | 70 |
| | Zinc laurate | | | | | | | | |
| | Sodium palmitate | | | | | | | | |
| | Calcium caprylate | | | | | | | | |
| | Sodium behenate | | | | | | | | |
| | Sodium oleate | | | | | | | | |
| | Calcium linoleate | | | | | | | | |
| Inorganic oxide internal addition amount | Titanium dioxide | 30 | 70 | 82 | 30 | 30 | 30 | 30 | 30 |
| | Zinc oxide | | | | | | | | |
| | Zeolite | | | | | | | | |
| Inorganic oxide external addition amount | Titanium dioxide | 4 | 7 | 7 | 1.5 | 1.5 | 1.5 | 1.5 | 1.5 |
| | Zinc oxide | | | | | | | | |
| | Zeolite | | | | | | | | |
| Inorganic oxide Total (weight%) | | 32.7 | 72.0 | 83.2 | 31.0 | 31.0 | 31.0 | 31.0 | 31.0 |
| Structure of core particle | Circularity | 0.95 | 0.95 | 0.95 | 0.95 | 1.95 | 2.95 | 0.95 | 0.95 |
| | D50 ($\mu$m) | 9 | 9 | 9 | 13 | 18 | 23 | 9 | 9 |
| Structure of inorganic oxide | Average particle size (μm) | 0.3 | 0.3 | 0.3 | 0.3 | 0.3 | 0.3 | 0.7 | 0.9 |
| | Hydrophobic treatment | SiCp | SiCp | SiCp | SiCp | SiCp | SiCp | SiCp | SiCp |
| Evaluation | Skin feel | ◎ | ○ | × | ◎ | ○ | △ | ◎ | ○ |
| | Durability of function | ◎ | ◎ | ○ | ◎ | ◎ | △ | ◎ | △ |
| | Overall | ◎ | ○ | × | ◎ | ○ | △ | ◎ | △ |

In Examples 1 to 27, the content of inorganic oxide in the soap composite particles was 5 to 80% by weight, and the shape of the core particles was spherical with a circularity of 0.80 to 1.00. The overall evaluation was given as A, B, or C.

Comparative Examples 1 to 5 were given an overall rating of D.

In Comparative Example 1, the circularity of the core particles was less than 0.80, but the evaluation of skin feel was D.

In Comparative Example 2, the content of inorganic oxide in the soap composite particles was less than 5% by weight, but the evaluation of the durability of function was D.

In Comparative Examples 3 and 4, an unsaturated fatty acid salt was used in place of the saturated higher fatty acid salt, but the evaluation of the skin feel was D.

In Comparative Example 5, the content of inorganic oxide in the soap composite particles was more than 80% by weight, but the evaluation of the skin feel was D.

INDUSTRIAL APPLICABILITY

The soap composite particles of the present invention can achieve both an improvement in skin feel and durability of the inorganic oxide function, and can be used in the production of cosmetics.

**Claims**

1.  Soap composite particles comprising:

14

a core particle containing a saturated higher fatty acid salt and an inorganic oxide, and an inorganic oxide externally added to the core particle,

**characterized in that** the content of the inorganic oxide in the soap composite particles is 5 to 80% by weight, and the shape of the core particle is spherical with a circularity of 0.80 to 1.00.

2. The soap composite particles according to claim 1, **characterized in that** the carbon number of the saturated higher fatty acid salt is within the range of 10 to 20.

3. The soap composite particles according to claim 1, **characterized in that** the metal forming the saturated higher fatty acid salt is one or more of Li, Na, Al, Mg, Ca, Zn and Ba.

4. The soap composite particles according to claim 1, **characterized in that** the content of the inorganic oxide in the core particle is 5 to 75% by weight, and the amount of the inorganic oxide added externally per 100 parts by weight of the core particle is 0.01 to 5.0 parts by weight.

5. The soap composite particles according to claim 1, **characterized in that** the inorganic oxide is one or more of zinc oxide, magnesium oxide, titanium dioxide, mica, talc, kaolin, sericite, silica, iron oxide, alumina, zirconia, chromium oxide, and zeolite.

6. The soap composite particles according to claim 1, **characterized in that** the volume-based median diameter of the core particle is greater than 3 $\mu$m and less than 20 $\mu$m.

7. The soap composite particles according to claim 1, **characterized in that** the inorganic oxide is subjected to a hydrophobic treatment.

8. The soap composite particles according to claim 7, **characterized in that** the hydrophobic treatment is any one of a silane coupling treatment, a silicone oil treatment, and a fatty acid salt treatment.

9. The soap composite particle according to claim 1, **characterized in that** the inorganic oxide has an average particle size of 0.01 to 0.8 $\mu$m.

10. A method for producing the soap composite particles according to any one of claims 1 to 9, comprising:

    a kneading step of melting and kneading a mixed powder of a saturated higher fatty acid salt and an inorganic oxide;
    a pulverizing step of pulverizing the solid kneaded product obtained in the kneading step;
    a spheroidization step of heating the particles obtained in the pulverizing step to form them into spheroids; and
    an external addition step of externally adding an inorganic oxide to core particles obtained in the spheroidization step.

11. A cosmetic comprising the soap composite particles according to any one of claims 1 to 9.

## INTERNATIONAL SEARCH REPORT

| International application No. |
| --- |
| **PCT/JP2023/038472** |

| | |
| --- | --- |
| **A.** | **CLASSIFICATION OF SUBJECT MATTER** |

*C07C 53/126*(2006.01)i; *C11D 17/06*(2006.01)i; *A61Q 19/10*(2006.01)i; *C11D 1/04*(2006.01)i; *C11D 3/12*(2006.01)i;
*A61K 8/19*(2006.01)i; *A61K 8/26*(2006.01)i; *A61K 8/29*(2006.01)i; *A61K 8/36*(2006.01)i
FI: C07C53/126 CSP; A61K8/36; A61K8/19; A61K8/29; A61K8/26; A61Q19/10; C11D17/06; C11D1/04; C11D3/12

According to International Patent Classification (IPC) or to both national classification and IPC

| | |
| --- | --- |
| **B.** | **FIELDS SEARCHED** |

Minimum documentation searched (classification system followed by classification symbols)

C07C53/126; C11D1/00-19/00; A61K8/00-8/99; A61Q1/00-90/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| A | WO 2016/132967 A1 (NOF CORPORATON) 25 August 2016 (2016-08-25) | 1-11 |
| A | JP 2011-6362 A (LION CORPORATION) 13 January 2011 (2011-01-13) | 1-11 |
| A | JP 61-257907 A (POLA CHEMICAL INDUSTRIES, INC.) 15 November 1986 (1986-11-15) | 1-11 |
| A | WO 2017/057675 A1 (SHISEIDO CO., LTD.) 06 April 2017 (2017-04-06) | 1-11 |
| A | KR 10-2012-0026824 A (HA, Seung Joon) 20 March 2012 (2012-03-20) | 1-11 |

☐ Further documents are listed in the continuation of Box C.  ☑ See patent family annex.

| | |
| --- | --- |
| \* | Special categories of cited documents: |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **26 December 2023** | **16 January 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/038472**

| Patent document cited in search report | | | Publication date (day/month/year) | Patent family member(s) | | | Publication date (day/month/year) |
|---|---|---|---|---|---|---|---|
| WO | 2016/132967 | A1 | 25 August 2016 | US | 2018/0093938 | A1 | |
| | | | | EP | 3260440 | A1 | |
| | | | | CN | 107207400 | A | |
| | | | | KR | 10-2017-0118060 | A | |
| JP | 2011-6362 | A | 13 January 2011 | (Family: none) | | | |
| JP | 61-257907 | A | 15 November 1986 | (Family: none) | | | |
| WO | 2017/057675 | A1 | 06 April 2017 | US | 2018/0271757 | A1 | |
| | | | | EP | 3357483 | A1 | |
| | | | | KR | 10-2018-0050421 | A | |
| | | | | CN | 108289811 | A | |
| KR | 10-2012-0026824 | A | 20 March 2012 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 6729546 A